# EUROPÄISCHE PATENTSCHRIFT

(11) **EP 0 551 614 B1**
(45) Veröffentlichungstag und Bekanntmachung des Hinweises auf die Patenterteilung: **10.04.1996**
(21) Anmeldenummer: 92121027.4
(22) Anmeldetag: 10.12.1992
(51) Int. Cl.: C12P 13/08, C12N 1/20

(54) **Verfahren zur Erhöhung der Leistungsfähigkeit L-Lysin ausscheidender coryneformer Bakterien**
Process for the enhancement of the performance of L-lysine secreting coryneform bacteria
Procédé d'augmentation de la productivité de bactéries corynéformes dégageant la L-lysine

(30) Priorität: 17.01.1992 DE 4201085
(43) Veröffentlichungstag der Anmeldung: 21.07.1993
(73) Patentinhaber: Degussa Aktiengesellschaft, D-60311 Frankfurt (DE)
(72) Erfinder: Kircher, Manfred, Dr., W-4800 Bielefeld 1 (DE); Bachmann, Bernd, Dr., W-4806 Werther (DE)

(56) Entgegenhaltungen:
- FR-A- 2 357 644

## Beschreibung

Die Erfindung betrifft ein Verfahren zur Erhöhung der Leistungsfähigkeit L-Lysin ausscheidender coryneformer Bakterien.

Die essentielle Aminosäure L-Lysin ist als Nahrungs- und Tierfutterzusatz, sowie als Wirkstoff und Bestandteil von pharmazeutischen Produkten Von großer industrieller Bedeutung.
Für die Herstellung von L-Lysin ist die Fermentation das bedeutendste Verfahren. Vor allem coryneforme Bakterien der Gattungen Corynebacterium und Brevibacterium werden in der Produktion eingesetzt. Durch Mutationen ist die Regulation der Lysin-Biosynthese dieser Stämme so verändert, daß sie Lysin über den Eigenbedarf hinaus produzieren und in das Medium ausscheiden.
Derartige Überproduzenten erhält man durch Suche nach Mutanten, in denen einzelne Schritte des Aminosäurestoffwechsels blockiert sind (z. B. Hse- oder Thr-Auxotrophe), die gegen ein oder mehrere Analoga von Lysin resistent sind oder die weitere Mutationen enthalten. Hochleistungsstämme besitzen im allgemeinen mehrere Auxotrophien, Analoga-Resistenzen oder eine Kombination von Mutationen. Eine zusammenfasssende Darstellung der Entwicklung von Lysin-Produzenten geben O. Tosaka und K. Takinami (Progr. Ind. Microbiol.(Biotechnol. Amino Acids) 24 (1986), 152-172; M. Hilliger, Biotec 2 (1991) 40-44.

Die Suche nach Mutanten, die L-Lysin produzieren, wird als Screening bezeichnet.

Im Screening werden in einem Ausgangsstamm mittels gebräuchlicher chemischer oder physikalischer Mutagene (z. B. MNNG oder UV) zufällige Mutationen induziert und mit üblichen mikrobiologischen Methoden Mutanten selektioniert. Entscheidend für den Erfolg des Screening ist nun die Auswahl des Selektionsmittels und dessen geeignete Anwendung.

Für die Selektion von Lysinproduzenten werden häufig Strukturanaloga von Lysin eingesetzt. Die das Wachstum hemmende Wirkung dieser Analoga wird durch L-Lysin aufgehoben. Unter Mutanten, die gegen das Analogon resistent sind, findet man deshalb auch solche mit erhöhter L-Lysinproduktion.
Ein bekanntes Beispiel für ein solches Strukturanalogon von L-Lysin ist AEG (S-[2-Aminoethyl]-Cystein). AEG unterscheidet sich von L-Lysin nur dadurch, daß in Position 4 das Kohlenstoffatom gegen ein Schwefelatom ausgetauscht ist. Dieses Analogon ist seit langem bekannt, und AEC-resistente Lysinproduzenten sind in der Literatur beschrieben (H. Kase, K. Nakayama; Agric. Biol. Chem. 38 (1974), 993 bis 1000; S.N. Kara-Murza et al.; Prikladnaya Biokhimiya Mikrobiologiya 16 (1980) 868 bis 875; US-PS 3,707,441).

Die Leistungsfähigkeit dieser Mutanten läßt sich durch Einführung weiterer Mutationen steigern. Bekannt ist die Kombination mit Auxotrophien, die sich mit dem Fachmann geläufigen Methoden leicht induzieren lassen (US-PS 3,708,395; US-PS 3,825,472; J. Plachy, Acta Biotechnol. 9 (1989) 3, 291-293; A. Sassi et al.; Biotechnol. Letter 12 (1990) 4, 295-298).

Weiterhin ist die Kombination mit weiteren Resistenzen bekannt. Beschrieben ist z. B. die Resistenz gegen Antibiotika (DE-OS 27 30964.)

Aufgaben der Erfindung ist es, die Leistungsfähigkeit Aminosäuren-, insbesondere L-Lysin-, ausscheidender coryneformer Bakterien durch geeignete Mutationen zu steigern und die entsprechenden Stämme durch ein Screening herauszufinden und zu charakterisieren.

Gegenstand der Erfindung ist ein Verfahren zur Erhöhung der Leistungsfähigkeit von L-Lysin ausscheidenden coryneformen Stämmen von Mikroorganismen, das dadurch gekennzeichnet ist, daß man bei diesen Stämmen eine Resistenz gegen L-Asparaginsäure-β-Methylester (AME) induziert.

Dies erfolgt in der Weise, daß der Ausgangsstamm gebräuchlichen chemischen oder physikalischen Mutagenen ausgesetzt wird, z. B. MNNG:
N-Methyl-N'-Nitro-N-Nitrosoguanidin oder UV-Strahlung. Die Selektion der geeigneten coryneformen Bakterien, die bevorzugt den Gattungen Corynebacterium und Brevibacterium, insbesondere Corynebacterium glutamicum, angehören, erfolgt nach allgemein bekannten mikrobiologischen Methoden.
Die so hergestellten und aufgefundenen Stämme sind ebenfalls Gegenstand der Anmeldung.

Im Gegensatz zu anderen Analoga der L-Asparaginsäure hemmt AME sowohl das Wachstum z. B. des Wildtyps von Corynebacterium glutamicum (ATTC13032) als auch das der davon abgeleiteten Mutanten.

Die eingesetzten Stämme können daneben weitere Resistenzen oder Auxotrophien aufweisen.

Die Fermentation zur Herstellung von L-Lysin erfolgt nach den allgemein bekannten Verfahren.

Die Tatsache, daß Mutanten, die bereits Lysin produzieren und deshalb eine der Lysinüberproduktion äquivalent gesteigerte Menge an Asparaginsäure synthetisieren, durch AME gehemmt werden, überrascht an sich.
Um so erstaunlicher ist im vorliegenden Fall, daß Mutanten, die gegen AME selektioniert werden, zusätzlich eine gegenüber dem Ausgangsstamm gesteigerte Lysinproduktion aufweisen.
Besonders geeignet sind erfindungsgemäß erhaltene AME-resitente Mutanten mit im Vergleich zu den Eltern-Stämmen reduziertem Citrat-Synthase-Gehalt. Diese Stammeigenschaft ist nach Literaturaussagen vorteilhaft für die Verbeserung der Ausscheidung von Aspartat-Aminosäuren, wie z. B. L-Lysin (A. YOTOKTA, J. SHIIO, Agric. Biol. Chem. 52, 455-463 (1988)). Die Bestimmung der Citrat-Synthase-Aktivität erfolgt nach P.A. SRERE et al. (Acta Chem. Scan 17, 129 (1963).

### Beispiele

Die Beispiele beziehen sich auf durch Behandlung mit MNNG erzeugte Mutanten von Corynebacterium glutamicum (ATCC13032).

### Beispiel 1

DM290-2 (hse⁻, AEC^{r}) wird über Nacht in Standard I Bouillon (Merck Art. 7882) angezogen, gegen physiologische Kochsalzlösung (0,9 % NaCl) gewaschen, mutagenisiert und auf Platten, die AME enthalten, ausgespatelt. Die Platten enthalten das Medium BMCG (Liebl et al., Appl. Microbiol. Biotechnol (1989) 32: 205-210) supplementiert mit 200 µg/ml D,L-Homoserin und 2 bis 24, vorzugsweise 4 bis 8 g/l AME. Nach 5 Tagen Inkubation bei 30 ⁰C werden resistente Kolonien abgeimpft.
Zur Prüfung der Lysinproduktion werden resistente Kolonien in CASO-Bouillon (Merck Art. 5459) 16 h inkubiert (300 rpm, 30 ⁰C). Diese Suspension wird 1:10 in 9 ml eines Mediums mit 240 g/l Melasse, 100 ml/l Sojamehlhydrolysat, 12 g/l Ammoniumsulfat, 10 g/l Calciumbarbonat (pH = 7) in 100 ml-Erlenmeyerkolben mit Schikane verdünnt und 48 h inkubiert (30 ⁰C, 300 rpm). Nach 48 h wird die Fermentationsbrühe abzentrifugiert und die Lysinkonzentration im Überstand mittels Aminosäureanalyse bestimmt.
Zur Ermittlung der spezifischen Citrat-Synthase-Aktivität werden die Stämme in Standard I Boullion (Merk Art. 7881) und 4 g/l Glucose kultiviert. Die Ernte der Zellen und die Herstellung der Enzympräparation wird nach einer beschriebenen Methode durchgeführt (G. THIERBACH et al., Appl. Microbiol. Biotechnol. 32, 443-448 (1990))

| Stamm | Phänotyp | Lys*HCl g/l] | Citrat-Synthase [U/mg] |
|---|---|---|---|
| DM290-2 | hse⁻, AEC^{r} | 36,5 | 0,156 |
| DM599 | hse⁻, AEC^{r}, AME^{r} | 40.0 | 0,126 |
| DM601 | hse⁻, AEC^{r}, AME^{r} | 42,8 | 0,105 |

### Beispiel 2

DM282-2 (leu⁻, AEC^{r)} wird über Nacht in Standard I Bouillon (Merck Art. 7882) angezogen, gegen physiologische Kochsalzlösung (0,9 % NaCl) gewaschen, mutagenisiert und auf Platten, die AME enthalten, ausgespatelt. Die Platten enthalten das Medium BMCG, supplementiert mit 100 µg/ml L-Leucin und AME wie in Beispiel 1.
Nach 5 Tagen Inkubation bei 30 ⁰C werden resistente Kolonien abgeimpft.
Die Prüfung der Lysinproduktion erfolgt wie in Beispiel 1 in einem Medium mit Melasse 30 g/l, Saccharose 85 g/l, Sojamehlhydrolysat 158 g/l, L-Leu 100 mg/l, Ammoniumsulfat 25 g/l, Kaliumhydrogenphosphat 0,5 g/l, Magnesiumsulfat 0,4 g/l, Calciumchlorid 10 mg/l, Eisensulfat 12 mg/l, Mangansulfat 11 mg/l, Citrat 0,6 g/l, Biotin 0,3 mg/l,Thiamin 0,2 mg/l, Calciumcarbonat 25 g/l Die Ermittlung der spezifischen Citrat-Synthase-Aktivität erfolgt wie in Beispiel 1 beschrieben.

| Stamm | Phänotyp | Lys*HCl [g/l] | Citrat-Synthase [U/mg] |
|---|---|---|---|
| DM282-2 | leu⁻, AEC^{r} | 29,9 | 1,02 |
| DM597 | leu⁻, AEC^{r}, AME^{r} | 34,4 | 0,957 |
| DM596 | leu⁻, AEC^{r}, AME^{r} | 33,2 | 0,983 |

### Beispiel 3

DM286-1 (hse⁻, leu⁻, Pen^{r}, AEC^{r}) wird über Nacht in Standard I Bouillon (Merck Art. 7882) angezogen, gegen physiologische Kochsalzlösung (0,9 % NaCl) gewaschen, mutagenisiert und auf Platten, die AME enthalten, ausgespatelt. Die Platten enthalten das Medium BMCG, supplementiert mit 100 µg/ml L-Leucin und 160 µg/ml DL-Homoserin und AME wie in Beispiel 1. Nach 5 Tagen Inkubation bei 30 ⁰C werden resistente Kolonien abgeimpft.
Die Prüfung der Lysinproduktion und die Bestimmung der spezifischen Citrat-Synthase-Aktivität erfolgt wie in Beispiel 2:

| Stamm | Phänotyp | Lys*HCl [g/l] | Citrat-Synthase [U/mg] |
|---|---|---|---|
| DM286-1 | hse^{r}, leu⁻, Pen^{r}, AEC^{r} | 36,1 | 0,968 |
| DM608 | hse^{r}, leu⁻, Pen^{r}, AEC^{r}, AME^{r}, | 37,0 | 0,098 |
| DM607, | hse^{r}, leu⁻, Pen^{r}, AEC^{r}, AME^{r} | 39,5 | 0,120 |

### Beispiel 4

Hemmzone von Corynebacterium glutamicum (ATCC13032) in Abhängigkeit von AME

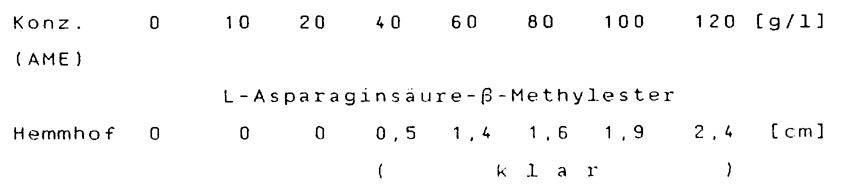

Eine Zellsuspension wird in Weichagar (BMCG) eingegossen. Nach Erstarrung werden 0,15 ml einer AME-Lösung in MOPS-Puffer (0,1 M, pH = 7) in einen Stahlzylinder (d = 0,5 cm) auf dem Agar getropft. Nach 3 Tagen Inkubation (30 ⁰C) wird der Hemmhof gemessen und beurteilt.

## Patentansprüche

1. Verfahren zur Erhöhung der Leistungsfähigkeit von L-Lysin ausscheidenden coryneformen Stämmen von Mikroorganismen,
dadurch gekennzeichnet, daß man bei diesen Stämmen eine Resistenz gegen L-Asparaginsäure-β-Methylester induziert.

2. Verfahren gemäß Anspruch 1
dadurch gekennzeichnet, daß die aus diesen Stämmen herrührenden Mutanten zusätzlich eine geringere Citrat-Synthese-Aktivität als die Eltern-Stämme besitzen.

3. Verfahren gemäß den Ansprüchen 1 oder 2,
dadurch gekennzeichnet, daß man Stämme der Gattungen Corynebacterium oder Brevibacterium einsetzt.

4. L-Lysin ausscheidende Stämme der Gattungen Corynebacterium oder Brevibacterium hergestellt nach Anspruch 1, die eine Resistenz gegen L-Asparaginsäure-β-Methylester aufweisen.

5. L-Lysin ausscheidende Stämme, hergestellt nach Anspruch 2, die zusätzlich eine geringere Citrat-Synthese-Aktivität als die Eltern-Stämme besitzen.

6. Verwendung der L-Lysin ausscheidenden Stämme gemäß den Ansprüchen 4 oder 5 zur fermentativen Herstellung von L-Lysin.

## Claims

1. Process for enhancing the performance of coryneform strains of micro-organisms which secrete L-lysine, characterised in that a resistance to L-aspartic acid β-methyl ester is induced in these strains.

2. Process according to Claim 1,
characterised in that the mutants stemming from these strains additionally possess a lower citrate-synthesis activity than the parent strains.

3. Process according to Claim 1 or 2,
characterised in that use is made of strains of the genera Corynebacterium or Brevibacterium.

4. Strains of the genera Corynebacterium or Brevibacterium which secrete L-lysine produced according to Claim 1 which exhibit a resistance to L-aspartic acid β-methyl ester.

5. Strains which secrete L-lysine produced according to Claim 2 which additionally possess a lower citrate-synthesis activity than the parent strains.

6. Use of the strains which secrete L-lysine according to Claims 4 or 5 for the fermentative production of L-lysine.

## Revendications

1. Procédé pour augmenter la productivité de souches corynéformes de micro-organismes produisant de la L-lysine, caractérisé en ce qu'on induit dans ces souches une résistance contre l'ester β-méthylique de l'acide L-aspartique.

2. Procédé selon la revendication 1, caractérisé en ce que les mutants qui proviennent de ces souches possèdent en outre une activité de synthèse de citrate inférieure à celle des souches-mères.

3. Procédé selon les revendications 1 ou 2, caractérisé en ce qu'on met en oeuvre des souches du type Corynebacterium ou Brevibacterium.

4. Souches de type Corynebacterium ou Brevibacterium qui produisent de la L-lysine produites selon la revendication 1, qui présentent une résistance contre l'ester β-méthylique de l'acide L-aspartique.

5. Souches produisant de la L-lysine, produites selon la revendication 2, qui possèdent en outre une activité de synthèse de citrate inférieure à celle des souches-mères.

6. Utilisation des souches produisant de la L-lysine selon les revendications 4 ou 5 pour la production par fermentation de L-lysine.
